(19) **Europäisches Patentamt** / **European Patent Office** / **Office européen des brevets**

(11) **EP 4 190 807 A1**

(12) **EUROPEAN PATENT APPLICATION**
published in accordance with Art. 153(4) EPC

(43) Date of publication:
07.06.2023 Bulletin 2023/23

(21) Application number: 21849139.7

(22) Date of filing: 30.07.2021

(51) International Patent Classification (IPC):
*C07K 14/715* (2006.01)   *C12N 15/12* (2006.01)
*C07K 19/00* (2006.01)   *C12N 15/62* (2006.01)
*C12N 15/867* (2006.01)   *C12N 5/10* (2006.01)
*A61K 39/00* (2006.01)   *A61P 35/00* (2006.01)

(86) International application number:
**PCT/CN2021/109864**

(87) International publication number:
**WO 2022/022719 (03.02.2022 Gazette 2022/05)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: 31.07.2020   CN 202010760960

(71) Applicant: **Beijing Neurosurgical Institute
Beijing 100070 (CN)**

(72) Inventors:
• **ZHANG, Wei
Beijing 100070 (CN)**

• **JIANG, Tao
Beijing 100070 (CN)**
• **ZHAI, You
Beijing 100070 (CN)**
• **LI, Guanzhang
Beijing 100070 (CN)**

(74) Representative: **Prüfer & Partner mbB
Patentanwälte · Rechtsanwälte
Sohnckestraße 12
81479 München (DE)**

Remarks:
The complete document including Reference Table(s) and the Sequence Listing(s) can be downloaded from the EPO website

(54) **TRUNCATED BODY OF IL7R ALPHA AND USE THEREOF IN PREPARATION OF MEDICATION FOR TREATING TUMOR**

(57) The present invention provides a truncated body of IL7Rα. The amino acid sequence of the truncated body of IL7Rα comprises a sequence shown in SEQ ID NO. 1. A T cell expressing a chimeric antigen receptor containing the truncated body of IL7Rα can effectively kill a tumor cell.

Fig. 1

**Description**

**TECHNICAL FIELD**

The disclosure relates to the field of pharmaceutical biotechnology, in particular to a truncated body of IL7Rα, an encoding nucleic acid, an expression vector, a cell, a pharmaceutical composition and use thereof.

**BACKGROUND**

Chimeric antigen receptor (CAR) is a synthetic T cell receptor consisting of an antigen binding domain, a transmembrane domain and an intracellular signaling domain. The antigen binding domain is located outside a T cell membrane, includes a single-chain antibody or ligand, and is used for specifically binding to a target antigen. The intracellular signaling domain is located in the T cell membrane, and is used for transmitting signals to a T cell to stimulate the immune response of the T cell.

The CAR can target and identify the target antigen on the surface of a tumor cell, so the T cell expressing the CAR can be used for targeting and killing a tumor cell. However, the existing T cell expressing the CAR is still weak in killing the tumor cell.

**SUMMARY**

The objective of the disclosure is to overcome the problem that the existing T cell expressing a chimeric antigen receptor (CAR) is still weak in killing a tumor cell, and to provide a truncated body of IL7Rα.

In order to achieve the above objective, in a first aspect, the disclosure provides a truncated body of IL7Rα, the amino acid sequence of which includes a sequence shown in SEQ ID NO. 1.

In a second aspect, the disclosure provides a nucleic acid encoding the truncated body of IL7Rα according to the first aspect; and preferably, the nucleic acid has a nucleotide sequence shown in SEQ ID NO. 2.

In a third aspect, the disclosure provides a fusion protein containing an antigen binding domain, a transmembrane domain and an intracellular signaling domain that are sequentially linked; the amino acid sequence of the intracellular signaling domain includes the amino acid sequence of the truncated body of IL7Rα according to the first aspect; preferably, the antigen binding domain includes an anti-CD44 single-chain antibody and/or an anti-CD133 single-chain antibody, and the intracellular signaling domain includes the truncated body of IL7Rα; and more preferably, the fusion protein has an amino acid sequence shown in SEQ ID NO. 3.

In a fourth aspect, the disclosure provides a fusion nucleic acid encoding the fusion protein according to the third aspect; and preferably, the fusion nucleic acid has a nucleotide sequence shown in SEQ ID NO. 4.

In a fifth aspect, the disclosure provides an expression vector, the expression vector is inserted with an expression cassette, the expression cassette includes a first nucleic acid fragment encoding an antigen binding molecule and a second nucleic acid fragment encoding an intracellular signaling molecule, the intracellular signaling molecule contains the truncated body of IL7Rα according to the first aspect, and an IRES element or a 2A peptide encoding sequence is inserted between the first nucleic acid fragment and the second nucleic acid fragment; and preferably, the expression cassette is the fusion nucleic acid according to the fourth aspect.

In a sixth aspect, the disclosure provides a cell expressing a chimeric antigen receptor, the cell expressing a chimeric antigen receptor is obtained by transfection of the expression vector according to the fifth aspect by a host cell, and the chimeric antigen receptor contains the truncated body of IL7Rα according to the first aspect.

Optionally, the host cell is a T cell.

In a seventh aspect, the disclosure provides use of the truncated body of IL7Rα according to the first aspect, the nucleic acid according to the second aspect, the fusion protein according to the third aspect, the fusion nucleic acid according to the fourth aspect, the expression vector according to the fifth aspect, and the cell expressing a chimeric antigen receptor according to the sixth aspect in preparation of a medication for treating a tumor.

Optionally, the tumor is glioma.

In an eighth aspect, the disclosure provides a pharmaceutical composition, an active ingredient of which includes the cell expressing a chimeric antigen receptor containing the truncated body of IL7Rα according to the sixth aspect.

Through the above technical solution, a T cell expressing a chimeric antigen receptor containing the truncated body of IL7Rα provided by the disclosure can effectively kill a tumor cell.

Other features and advantages of the disclosure will be described in detail in the following specific embodiments.

**BRIEF DESCRIPTION OF THE DRAWINGS**

The accompanying drawings are intended to provide a further understanding of the disclosure, constitute a part of the description, and are used for interpreting the disclosure together with the following specific embodiments, rather than limiting the disclosure. In the figures:

Fig. 1 is a flow cytometer test result diagram of CAR-T 1 cells according to an embodiment of the disclosure;
Fig. 2 is a flow cytometry test result diagram of CAR-T 2 cells provided by an embodiment of the disclosure;
Fig. 3 is a flow cytometry test result diagram of CAR-T 3 cells provided by an embodiment of the disclosure; and
Fig. 4 is a flow cytometer test result diagram of CAR-T 4 cells according to an embodiment of the disclosure.

**DETAILED DESCRIPTION**

The specific embodiments of the disclosure will be described in detail below with reference to the accompanying drawings. It should be understood that the specific embodiments described herein are merely used for illustrating and interpreting the disclosure, rather than limiting the disclosure.

A first aspect of the disclosure provides a truncated body of IL7Rα. The amino acid sequence of the truncated body of IL7Rα includes a sequence shown in SEQ ID NO. 1.

The sequence shown in SEQ ID NO. 1 is as follows:

KKRIKPIVWPSLPDHKKTLEHLCKKPRKNLNVSFNPESFLDCQIHRVDDIQARD
EVEGFLQDTFPQQLEESEKQRLLGSNQEEAYVTMSSFYQNQ.

The inventors of the disclosure found that the above-mentioned truncated body of IL7Rα can effectively prolong the survival time of an activated T cell. Therefore, a T cell expressing a chimeric antigen receptor containing the truncated body of IL7Rα has a long survival time and can effectively kill a tumor cell.

A second aspect of the disclosure provides a nucleic acid encoding the truncated body of IL7Rα according to the first aspect. Preferably, the nucleic acid has a nucleotide sequence shown in SEQ ID NO. 2.

The nucleotide sequence shown in SEQ ID NO. 2 is as follows:

aaaaaaaggattaagcctatcgtatggcccagtctccccgatcacaagaagactctggaacacctttgtaagaaaccaagaa
aaaatttaaatgtgagtttcaatcctgaaagtttcctggactgccagattcatagggtggatgacattcaagctagagatgaagtggaaggttt
tctgcaagatacgtttcctcagcaactagaagaatctgagaagcagaggcttctgggatcaaatcaagaagaagcatatgtcaccatgtcc
agcttctaccaaaaccag.

A third aspect of the disclosure provides a fusion protein containing an antigen binding domain, a transmembrane domain and an intracellular signaling domain that are sequentially linked. The amino acid sequence of the intracellular signaling domain includes the amino acid sequence of the truncated body of IL7Rα according to the first aspect. Preferably, the antigen binding domain includes an anti-CD44 single-chain antibody and/or an anti-CD133 single-chain antibody, and the intracellular signaling domain includes the truncated body of IL7Rα. More preferably, the fusion protein has an amino acid sequence shown in SEQ ID NO. 3.

The fusion protein shown in SEQ ID NO. 3 consists of an anti-CD44 single-chain antibody, an anti-CD 133 single-chain antibody, a CD28, a truncated body of IL7Rα, and a CD3.

The amino acid sequence shown in SEQ ID NO. 3 is as follows:

QVQLVESGGGVVQPGRSLRLSCAASGFTFSSYGMHWVRQAPGKGLEWVAVI
WYDGSNKFYADSVKGRFTISRDNSKNTLYLQMNSLRAEDTAVYYCARRSDYRGYYGM
DVWGQGTTVTVSSGSTSGGGSGGGSGGGGSSEIVLTQSPATLSLSPGERATLSCRASQSV
INYLAWYQQKPGQAPRLLIYDASNRASGIPARFSGSGSGTDFTLTISSLEPEDFAVYYCQQ
RRNWPLTFGGGTKVEIKGGGGSGGGGSGGGGSQVQLVQSGAEVKKPGASVKVSCKAS
GYTFTDFEMHWVRQAPGQGLEWMGDIDPGTGDTAYNLKFKGRVTMTTDTSTSTAYME
LRSLRSDDTAVYYCALGAFVYWGQGTLVTVSSGSTSGGGSGGGSGGGGSSDVVMTQS
PLSLPVTPGEPASISCRSSQSLANSYGNTYLSWYLQKPGQSPQLLIYGISNRFSGVPDRFS
GSGSGTDFTLKISRVEAEDVGVYYCLQGTHQPYTFGQGTKLEIKTTTPAPRPPTPAPTIAS
QPLSLRPEACRPAAGGAVHTRGLDFACDIYIWAPLAGTCGVLLLSLVITLYCRSKRSRLL
HSDYMNMTPRRPGPTRKHYQPYAPPRDFAAYRSKKRIKPIVWPSLPDHKKTLEHLCKKP
RKNLNVSFNPESFLDCQIHRVDDIQARDEVEGFLQDTFPQQLEESEKQRLLGSNQEEAY
VTMSSFYQNQRVKFSRSADAPAYKQGQNQLYNELNLGRREEYDVLDKRRGRDPEMGG
KPRRKNPQEGLYNELQKDKMAEAYSEIGMKGERRRGKGHDGLYQGLSTATKDTYDAL
HMQALPPR.

A fourth aspect of the disclosure provides a fusion nucleic acid encoding the fusion protein according to the third aspect. Preferably, the fusion nucleic acid has a nucleotide sequence shown in SEQ ID NO. 4.

The nucleotide sequence shown in SEQ ID NO. 4 is used for encoding the amino acid sequence shown in SEQ ID NO. 3.

The nucleotide sequence shown in SEQ ID NO. 4 is as follows:

caggtgcagctggtggagtctggggggaggcgtggtccagcctgggaggtccctgagactctcctgtgcagcgtctggattc
accttcagtagctatggcatgcactgggtccgccaggctccaggcaaggggctggagtgggtggcagttatatggtatgatggaagtaat
aaattctatgcagactccgtgaagggccgattcaccatctccagagacaattccaagaacacgctgtatctgcaaatgaacagcctgagag
ccgaggacacggctgtgtattactgtgcgaggagaagtgactacaggggctactacggtatggacgtctggggccaagggaccacggt
caccgtctcctcaggcagtactagcggtggtggctccggggggcggttccggtggggggcggcagcagcgaaattgtgttgacacagtctc
cagccaccctgtctttgtctccaggggaaagagccaccctctcctgcagggccagtcagagtgttatcaactacttagcctggtaccaaca
gaaacctggccaggctcccaggctcctcatctatgatgcatccaacagggcctctggcatcccagccaggttcagtggcagtgggtctgg

gacagacttcactctcaccatcagcagcctagagcctgaagattttgcagtttattactgtcagcagcgtcgcaactggccgctcactttcgg

cggagggaccaaggtggagatcaaaggaggtggtggatccggaggtggtggctccggaggtggtggatcccaggttcagctggtgca

gtctggagctgaggtgaagaagcctggggcctcagtgaaggtctcctgcaaggcttctggttacacctttaccgactttgaaatgcactgg

gtgcgacaggcccctggacaagggcttgagtggatgggagatattgatcctggaactggtgatactgcctacaatctgaagttcaagggc

agagtcaccatgaccacagacacatccacgagcacagcctacatggagctgaggagcctgaggtctgacgacacggccgtgtattactg

tgcgttggggggcctttgtttactggggccagggaaccctggtcaccgtctcctcaggcagtactagcggtggtggctccggggggcggttc

cggtggggggcggcagcagcgatgttgtgatgactcagtctccactctccctgcccgtcacccctggagagccggcctccatctcctgcag

gtctagtcagagtcttgcaaacagttatgggaacacctatttgtcttggtacctgcagaagccagggcagtctccacagctcctgatctatgg

gatttccaacagattttctggggtccctgacaggttcagtggcagtggatcaggcacagattttacactgaaaatcagcagagtggaggctg

aggacgttggggtttattactgcttacaaggtacacatcagccgtacacgtttggccaggggaccaagctggagatcaaaaccactacccc

agcaccgaggccacccaccccggctcctaccatcgcctcccagcctctgtccctgcgtccggaggcatgtagacccgcagctggtggg

gccgtgcatacccggggtcttgacttcgcctgcgatatctacatttgggcccctctggctggtacttgcggggtcctgctgctttcactcgtga

tcactctttactgtaggagtaagaggagcaggctcctgcacagtgactacatgaacatgactccccgccgccccgggcccacccgcaag

cattaccagccctatgccccaccacgcgacttcgcagcctatcgctccaaaaaaaggattaagcctatcgtatggcccagtctccccgatc

acaagaagactctggaacacctttgtaagaaaccaagaaaaaatttaaatgtgagtttcaatcctgaaagtttcctggactgccagattcata

gggtggatgacattcaagctagagatgaagtggaaggttttctgcaagatacgtttcctcagcaactagaagaatctgagaagcagaggct

tctgggatcaaatcaagaagaagcatatgtcaccatgtccagcttctaccaaaaccagcgcgtgaaattcagccgcagcgcagatgctcc

agcctacaagcaggggcagaaccagctctacaacgaactcaatcttggtcggagagaggagtacgacgtgctggacaagcggagagg

acgggacccagaaatgggcgggaagccgcgcagaaagaatccccaagagggcctgtacaacgagctccaaaaggataagatggcag

aagcctatagcgagattggtatgaaaggggaacgcagaagaggcaaaggccacgacggactgtaccagggactcagcaccgccacc

aaggacacctatgacgctcttcacatgcaggccctgccgcctcgg.

A fifth aspect of the disclosure provides an expression vector, the expression vector is inserted with an expression cassette, the expression cassette includes a first nucleic acid fragment encoding an antigen binding molecule and a second nucleic acid fragment encoding an intracellular signaling molecule, the intracellular signaling molecule contains the truncated body of IL7Rα according to the first aspect, and an IRES element or a 2A peptide coding sequence is inserted between the first nucleic acid fragment and the second nucleic acid fragment; and preferably, the expression cassette is the fusion nucleic acid according to the fourth aspect.

A sixth aspect of the disclosure provides a cell expressing a chimeric antigen receptor, the cell expressing a chimeric antigen receptor is obtained by transfection of the expression vector according to the fifth aspect by a host cell, and the chimeric antigen receptor contains the truncated body of IL7Rα according to the first aspect.

Optionally, the host cell is a T cell.

A seventh aspect of the disclosure provides use of the truncated body of IL7Rα according to the first aspect, the nucleic acid according to the second aspect, the fusion protein according to the third aspect, the fusion nucleic acid according to the fourth aspect, the expression vector according to the fifth aspect, and the cell expressing a chimeric antigen receptor according to the sixth aspect in preparation of a medication for treating a tumor.

Optionally, the tumor is glioma.

An eighth aspect of the disclosure provides a pharmaceutical composition, an active ingredient of which includes the cell expressing a chimeric antigen receptor containing the truncated body of IL7Rα according to the sixth aspect.

The disclosure is further described by the following examples, but the disclosure is not limited thereby.

Example 1

This example was used to explain the construction of expression vectors.

(1) Nucleotide sequences shown in SEQ ID NO. 4, SEQ ID NO. 5 and SEQ ID NO. 6 were respectively synthesized by using a full sequence synthesis method. The nucleotide sequence shown in SEQ ID NO. 4 was used for encoding a fusion protein shown in SEQ ID NO. 3. The nucleotide sequence shown in SEQ ID NO. 5 was used for encoding a fusion protein shown in SEQ ID NO. 7. The nucleotide sequence shown in SEQ ID NO. 6 was used for encoding a fusion protein shown in SEQ ID NO. 8. The compositions of the fusion proteins shown in SEQ ID NO. 3, SEQ ID NO. 7 and SEQ ID NO. 8 were as follows:

G1: the fusion protein shown in SEQ ID NO. 3 consisted of an anti-CD44 single-chain antibody, an anti-CD 133 single-chain antibody, a CD28, a truncated body of IL7Rα, and a CD3;
G2: the fusion protein shown in SEQ ID NO. 7 consisted of an anti-CD44 single-chain antibody, an anti-CD133 single-chain antibody, a CD28, and a CD3;
G3: the fusion protein shown in SEQ ID NO. 8 consisted of an anti-CD44 single-chain antibody, an anti-CD 133 single-chain antibody, a CD28, an IL7Rα, and a CD3.

The amino acid sequence of the fusion protein shown in SEQ ID NO. 7 was as follows:

QVQLVESGGGVVQPGRSLRLSCAASGFTFSSYGMHWVRQAPGKGLEWVAVI
WYDGSNKFYADSVKGRFTISRDNSKNTLYLQMNSLRAEDTAVYYCARRSDYRGYYGM
DVWGQGTTVTVSSGSTSGGGSGGGSGGGGSSEIVLTQSPATLSLSPGERATLSCRASQSV
INYLAWYQQKPGQAPRLLIYDASNRASGIPARFSGSGSGTDFTLTISSLEPEDFAVYYCQQ
RRNWPLTFGGGTKVEIKGGGGSGGGGSGGGGSQVQLVQSGAEVKKPGASVKVSCKAS
GYTFTDFEMHWVRQAPGQGLEWMGDIDPGTGDTAYNLKFKGRVTMTTDTSTSTAYME
LRSLRSDDTAVYYCALGAFVYWGQGTLVTVSSGSTSGGGSGGGSGGGGSSDVVMTQS
PLSLPVTPGEPASISCRSSQSLANSYGNTYLSWYLQKPGQSPQLLIYGISNRFSGVPDRFS
GSGSGTDFTLKISRVEAEDVGVYYCLQGTHQPYTFGQGTKLEIKTTTPAPRPPTPAPTIAS

QPLSLRPEACRPAAGGAVHTRGLDFACDIYIWAPLAGTCGVLLLSLVITLYCRSKRSRLL
HSDYMNMTPRRPGPTRKHYQPYAPPRDFAAYRSRVKFSRSADAPAYKQGQNQLYNELN
LGRREEYDVLDKRRGRDPEMGGKPRRKNPQEGLYNELQKDKMAEAYSEIGMKGERRR
GKGHDGLYQGLSTATKDTYDALHMQALPPR.

The nucleotide sequence shown in SEQ ID NO. 5 was as follows:

caggtgcagctggtggagtctggggggaggcgtggtccagcctggggaggtccctgagactctcctgtgcagcgtctggattc accttcagtagctatggcatgcactgggtccgccaggctccaggcaaggggctggagtgggtggcagttatatggtatgatggaagtaat aaattctatgcagactccgtgaagggccgattcaccatctccagagacaattccaagaacacgctgtatctgcaaatgaacagcctgagag ccgaggacacggctgtgtattactgtgcgaggagaagtgactacagggggctactacggtatggacgtctggggccaagggaccacggt caccgtctcctcaggcagtactagcggtggtggctccggggggcggttccggtggggggcggcagcagcgaaattgtgttgacacagtctc cagccaccctgtctttgtctccaggggaaagagccaccctctcctgcagggccagtcagagtgttatcaactacttagcctggtaccaaca gaaacctggccaggctcccaggctcctcatctatgatgcatccaacagggcctctggcatcccagccaggttcagtggcagtgggtctgg gacagacttcactctcaccatcagcagcctagagcctgaagattttgcagtttattactgtcagcagcgtcgcaactggccgctcactttcgg cggagggaccaaggtggagatcaaaggaggtggtggatccggaggtggtggctccggaggtggtggatcccaggttcagctggtgca gtctggagctgaggtgaagaagcctggggcctcagtgaaggtctcctgcaaggcttctggttacacctttaccgactttgaaatgcactgg gtgcgacaggcccctggacaagggcttgagtggatgggagatattgatcctggaactggtgatactgcctacaatctgaagttcaagggc agagtcaccatgaccacagacacatccacgagcacagcctacatggagctgaggagcctgaggtctgacgacacggccgtgtattactg tgcgttggggggcctttgtttactggggccagggaaccctggtcaccgtctcctcaggcagtactagcggtggtggctccggggggcggttc cggtggggggcggcagcagcgatgttgtgatgactcagtctccactctccctgcccgtcacccctggagagccggcctccatctcctgcag gtctagtcagagtcttgcaaacagttatgggaacacctatttgtcttggtacctgcagaagccagggcagtctccacagctcctgatctatgg gatttccaacagattttctggggtccctgacaggttcagtggcagtggatcaggcacagattttacactgaaaatcagcagagtggaggctg aggacgttggggtttattactgcttacaaggtacacatcagccgtacacgtttggccaggggaccaagctggagatcaaaaccactacccc agcaccgaggccacccaccccggctcctaccatcgcctcccagcctctgtccctgcgtccggaggcatgtagacccgcagctggtggg gccgtgcatacccggggtcttgacttcgcctgcgatatctacatttgggcccctctggctggtacttgcggggtcctgctgctttcactcgtga tcactctttactgtaggagtaagaggagcaggctcctgcacagtgactacatgaacatgactccccgccgccccgggcccacccgcaag cattaccagccctatgccccaccacgcgacttcgcagcctatcgctcccgcgtgaaattcagccgcagcgcagatgctccagcctacaag caggggcagaaccagctctacaacgaactcaatcttggtcggagagaggagtacgacgtgctggacaagcggagaggacgggaccc agaaatgggcgggaagccgcgcagaaagaatccccaagagggcctgtacaacgagctccaaaaggataagatggcagaagcctata gcgagattggtatgaaaggggaacgcagaagaggcaaaggccacgacggactgtaccagggactcagcaccgccaccaaggacacc tatgacgctcttcacatgcaggccctgccgcctcgg.

The amino acid sequence of the fusion protein shown in SEQ ID NO. 8 was as follows:

QVQLVESGGGVVQPGRSLRLSCAASGFTFSSYGMHWVRQAPGKGLEWVAVI WYDGSNKFYADSVKGRFTISRDNSKNTLYLQMNSLRAEDTAVYYCARRSDYRGYYGM DVWGQGTTVTVSSGSTSGGGSGGGSGGGGSSEIVLTQSPATLSLSPGERATLSCRASQSV

INYLAWYQQKPGQAPRLLIYDASNRASGIPARFSGSGSGTDFTLTISSLEPEDFAVYYCQQ
RRNWPLTFGGGTKVEIKGGGGSGGGGSGGGGSQVQLVQSGAEVKKPGASVKVSCKAS
GYTFTDFEMHWVRQAPGQGLEWMGDIDPGTGDTAYNLKFKGRVTMTTDTSTSTAYME
LRSLRSDDTAVYYCALGAFVYWGQGTLVTVSSGSTSGGGSGGGSGGGGSSDVVMTQS
PLSLPVTPGEPASISCRSSQSLANSYGNTYLSWYLQKPGQSPQLLIYGISNRFSGVPDRFS
GSGSGTDFTLKISRVEAEDVGVYYCLQGTHQPYTFGQGTKLEIKTTTPAPRPPTPAPTIAS
QPLSLRPEACRPAAGGAVHTRGLDFACDIYIWAPLAGTCGVLLLSLVITLYCRSKRSRLL
HSDYMNMTPRRPGPTRKHYQPYAPPRDFAAYRSKKRIKPIVWPSLPDHKKTLEHLCKKP
RKNLNVSFNPESFLDCQIHRVDDIQARDEVEGFLQDTFPQQLEESEKQRLGGDVQSPNC
PSEDVVITPESFGRDSSLTCLAGNVSACDAPILSPSRSLDCRESGKNGPHVYQDLLLSLG
TTNSTLPPPFSLQSGILTLNPVAQGQPILTSLGSNQEEAYVTMSSFYQNQRVKFSRSADAP
AYKQGQNQLYNELNLGRREEYDVLDKRRGRDPEMGGKPRKNPQEGLYNELQKDKMA
EAYSEIGMKGERRRGKGHDGLYQGLSTATKDTYDALHMQALPPR.

The nucleotide sequence shown in SEQ ID NO. 6 was as follows:

caggtgcagctggtggagtctggggggaggcgtggtccagcctggggaggtccctgagactctcctgtgcagcgtctggattc

accttcagtagctatggcatgcactgggtccgccaggctccaggcaaggggctggagtgggtggcagttatatggtatgatggaagtaat

aaattctatgcagactccgtgaagggccgattcaccatctccagagacaattccaagaacacgctgtatctgcaaatgaacagcctgagag

ccgaggacacggctgtgtattactgtgcgaggagaagtgactacaggggctactacggtatggacgtctggggccaagggaccacggt

caccgtctcctcaggcagtactagcggtggtggctccggggggcggttccggtggggggcggcagcagcgaaattgtgttgacacagtctc

cagccaccctgtctttgtctccaggggaaagagccaccctctcctgcagggccagtcagagtgttatcaactacttagcctggtaccaaca

gaaacctggccaggctcccaggctcctcatctatgatgcatccaacagggcctctggcatcccagccaggttcagtggcagtgggtctgg

gacagacttcactctcaccatcagcagcctagagcctgaagattttgcagtttattactgtcagcagcgtcgcaactggccgctcactttcgg

cggagggaccaaggtggagatcaaaggaggtggtggatccggaggtggtggctccggaggtggtggatcccaggttcagctggtgca

gtctggagctgaggtgaagaagcctggggcctcagtgaaggtctcctgcaaggcttctggttacacctttaccgactttgaaatgcactgg

gtgcgacaggcccctggacaagggcttgagtggatgggagatattgatcctggaactggtgatactgcctacaatctgaagttcaagggc

agagtcaccatgaccacagacacatccacgagcacagcctacatggagctgaggagcctgaggtctgacgacacggccgtgtattactg

tgcgttggggggcctttgtttactggggccagggaaccctggtcaccgtctcctcaggcagtactagcggtggtggctccggggggcggttc

cggtggggggcggcagcagcgatgttgtgatgactcagtctccactctccctgcccgtcacccctggagagccggcctccatctcctgcag

gtctagtcagagtcttgcaaacagttatgggaacacctatttgtcttggtacctgcagaagccagggcagtctccacagctcctgatctatgg

gatttccaacagattttctggggtccctgacaggttcagtggcagtggatcaggcacagattttacactgaaaatcagcagagtggaggctg

aggacgttggggtttattactgcttacaaggtacacatcagccgtacacgtttggccaggggaccaagctggagatcaaaaccactacccc

agcaccgaggccacccaccccggctcctaccatcgcctcccagcctctgtccctgcgtccggaggcatgtagacccgcagctggtggg

gccgtgcatacccggggtcttgacttcgcctgcgatatctacatttgggcccctctggctggtacttgcggggtcctgctgctttcactcgtga

tcactctttactgtaggagtaagaggagcaggctcctgcacagtgactacatgaacatgactccccgccgccccgggcccacccgcaag

cattaccagccctatgccccaccacgcgacttcgcagcctatcgctccaaaaaaaggattaagcctatcgtatggcccagtctccccgatc

ataagaagactctggaacatctttgtaagaaaccaagaaaaaatttaaatgtgagtttcaatcctgaaagtttcctggactgccagattcatag

ggtggatgacattcaagctagagatgaagtggaaggttttctgcaagatacgtttcctcagcaactagaagaatctgagaagcagaggctt

ggaggggatgtgcagagcccccaactgcccatctgaggatgtagtcatcactccagaaagctttggaagagattcatccctcacatgcctg

gctgggaatgtcagtgcatgtgacgcccctattctctccccttccaggtccctagactgcagggagagtggcaagaatgggcctcatgtgt

accaggacctcctgcttagccttgggactacaaacagcacgctgcccctccattttctctccaatctggaatcctgacattgaacccagttg

ctcagggtcagcccattcttacttccctgggatcaaatcaagaagaagcatatgtcaccatgtccagcttctaccaaaaccagcgcgtgaaa

ttcagccgcagcgcagatgctccagcctacaagcaggggcagaaccagctctacaacgaactcaatcttggtcggagagaggagtacg

acgtgctggacaagcggagaggacgggacccagaaatgggcgggaagccgcgcagaaagaatccccaagagggcctgtacaacga

gctccaaaaggataagatggcagaagcctatagcgagattggtatgaaaggggaacgcagaagaggcaaaggccacgacggactgta

ccagggactcagcaccgccaccaaggacacctatgacgctcttcacatgcaggccctgccgcctcgg.

(2) The three nucleotide sequences synthesized in step (1) were respectively inserted into pLUX-IRES-ΔNGFR (purchased from Clontech Company, article number 631982) as a vector to obtain three kinds of lentivirus expression vectors of this example.

Example 2

This example was used to explain the preparation and test of T cells expressing a chimeric antigen receptor.

(1) The three kinds of lentivirus expression vectors constructed in Example 1 and an empty pLUX-IRES-ΔNGFR vector were packaged with lentiviruses respectively, and then T cells were cultured in vitro, transfected and proliferated according to the following methods.
(2) T cells in blood were separated according to the following method: 1 mL of sterile PBS and 1 mL of blood were mixed evenly, then slowly added to an upper layer of a lymphocyte separation medium Ficoll, and centrifuged at 4°C and 400 g for 30 min, with acceleration and deceleration set to 0 respectively. After centrifugation, the upper plasma was removed, the middle white membrane cells were pipetted, PBS was added for re-suspension washing, and centrifugation was carried out for 10 min at 100 g, with normal acceleration and deceleration. After centrifugation, the upper washing solution was removed, 1 mL of 1640 + 10% FBS + 1% double antibody + $1 \times$ Glutamine medium was added to re-suspend cells, and then the cells were stimulated to proliferate by means of anti-human CD3/CD28 magnetic beads (purchased from Thermo Fisher company), where the concentration of re-suspended cells was $1 \times 10^6$ cells/mL and the amount of magnetic beads added was 100 $\mu$L. Then 100 IU/mL rhIL-2 (Peprotech) was added to stimulate culture for 2 days to obtain T cells.
(3) Transfection of lentiviruses was carried out according to the following method: the lentiviruses packaged in step (1) were respectively added to four parts of T cells separated above, and then polybrene with a final concentration of 6 $\mu$g/mL was added, followed by uniform mixing and centrifugation at 32°C and 800 g for 100 min. After centrifugation, the culture was continued in an incubator for 24 h. After the culture, the culture solution was centrifuged at 1500 rpm for 15 min, and the centrifuged cells were inoculated at a density of $1 \times 10^6$/mL into a culture plate and stimulated for culture with rhIL2-100 IU/mL. After that, the medium was changed every 2-3 days until 2-4 weeks, and transfected T lymphocytes CAR-T 1, CAR-T 2, CAR-T 3 and CAR-T 4 were obtained. CAR-T 1 was transfected with the nucleotide sequence shown in SEQ ID NO. 4 and could express the fusion protein shown in SEQ ID NO. 3; CAR-T 2 was transfected with the nucleotide sequence shown in SEQ ID NO. 5 and could express the fusion protein shown in SEQ ID NO. 7; CAR-T 3 was transfected with the nucleotide sequence shown in SEQ ID NO. 6 and could express the fusion protein shown in SEQ ID NO. 8; and CAR-T 4 was transfected with the empty vector.

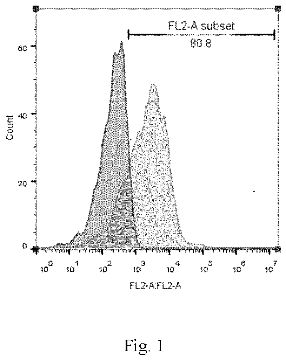

After culture, the cells were re-suspended with PBS, and the ratio of the above four kinds of transfected T lymphocytes and the expression of CAR protein on the surface were tested with a flow cytometer. The test method was as follows: the T cells to be tested after transfection were centrifuged and collected respectively, the supernatant was discarded after the T cells to be tested were washed with PBS once, and a corresponding test amount of monoclonal antibody was added according to antibody instructions and avoided light for 30min, followed by PBS washing, re-suspension, filtration with a membrane, and sandwich test with a flow cytometer, where the antibody used for the test was a mixture of His-tag labeled CD44 and PE labeled anti-His-tag antibody. The results were shown in Figs. 1-4.
Fig. 1 is a flow cytometer test result diagram of CAR-T 1 cells according to an embodiment of the disclosure;

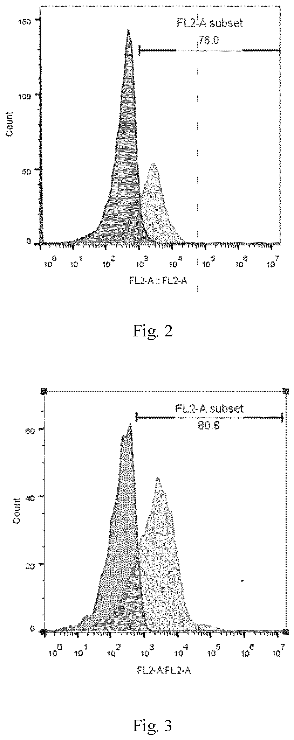

Fig. 2 is a flow cytometry test result diagram of CAR-T 2 cells according to an embodiment of the disclosure;
Fig. 3 is a flow cytometer test result diagram of CAR-T 3 cells according to an embodiment of the disclosure; and

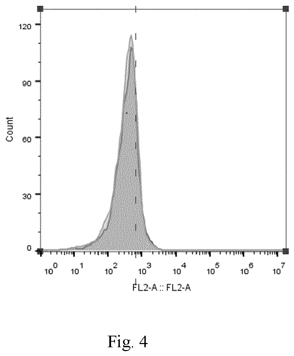

Fig. 4 is a flow cytometer test result diagram of CAR-T 4 cells according to an embodiment of the disclosure.
As can be seen from Figs. 1-3, other cells different from normal T lymphocytes were detected out from the CAR-T 1 cells, the CAR-T 2 cells and the CAR-T 3 cells. As can be seen from Fig. 4, other cells different from normal T lymphocytes were not detected out from the CAR-T 4 cells. This showed that the CAR-T cells transfected with fusion genes in this example had successfully expressed the target fusion protein.

Example 3

This example was used to verify the proliferation capacity and survival of the CAR-T cells constructed in Example 2.
Four kinds of CAR-T cells obtained by transfection and culture in Example 2 were respectively mixed with CD44 and CD133 positive glioma stem cells GSC20 according to an effect-target ratio of 10 (the number of T cells: the number of glioma stem cells). The mixed cells were cultured in 24-well plates, where each well contained $10^5$ glioma stem cells, and the reaction system was 1 $\mu$L per well. The culture conditions included: 37°C, 5% $CO_2$, and culture in a saturated humidity incubator. After 0, 4, 8, 12, 18, and 26 days, different CAR-T cells were counted with cell counting plates to explore the proliferation of different CAR-T cells stimulated by target cells.

Table 1

| CAR-T cells | Proliferation of different CAR-T cells stimulated by target cells ($\times 10^6$) | | | | | |
|---|---|---|---|---|---|---|
| | Day 0 | Day 4 | Day 8 | Day 12 | Day 18 | Day 26 |
| CAR-T 1 | 1.029 | 2.860 | 3.995 | 4.533 | 5.364 | 3.788 |
| CAR-T 2 | 1.147 | 2.435 | 3.151 | 3.080 | 0.665 | 0 |
| CAR-T 3 | 1.149 | 2.692 | 3.552 | 3.953 | 3.253 | 0 |
| CAR-T 4 | 1.135 | 2.494 | 2.999 | 0.464 | 0 | 0 |

As can be seen from Table 1, the CAR-T cells expressing the above-mentioned truncated body of IL7Rα had stronger proliferation capacity and longer survival.

Comparative Example

The CAR-T 1 cells obtained by transfection and culture in Example 2 and conventional second generation CAR-T cells (EGFR vIII-CD28-CD3) targeting a classical tumor target EGFR vIII were respectively mixed with CD44 positive and CD133 positive glioma stem cells GSC20 according to different effect-target ratios (the number of T cells: the number of glioma stem cells). The mixed cells were cultured in 96-well plates, where each well contained $4 \times 10^4$ glioma stem cells, and the reaction system was 200 μL per well. The culture conditions included: 37°C, 5% $CO_2$, and culture in a saturated humidity incubator for 4 h.

Determination of lactate dehydrogenase activity: after centrifugation, 100 μL of supernatant was aspirated from each well and placed in 96-well enzyme-labeled plates, 100 μL of LDH substrate was added to each well, and the reaction was carried out at room temperature for 30 min without light. After the reaction, 50 μL of termination solution was added to each well to terminate the enzymatic reaction. The optical density (OD) was measured at 490nm with a microplate reader. The average optical density (OD) of each group was calculated, and the lysis rate of glioma stem cells by each kind of T cells was calculated according to the following formula. The results were shown in Table 1.

Lysis rate% = (OD of the experimental group - OD spontaneously released by glioma stem cells - OD naturally released by effector cells)/ (maximum OD released by glioma stem cells - OD spontaneously released by glioma stem cells)

The test results were shown in Table 2.

Table 2

| | Lysis rate of glioma stem cells by conventional second generation CAR-T cells under different effect-target ratios (%) | | | |
|---|---|---|---|---|
| Effect-target ratio | 5:1 | 3:1 | 2:1 | 1:1 |
| CAR-T 1 | 84.74% | 67.20% | 37.13% | 31.48% |
| Conventional CAR-T | 17.57% | 14.62% | 13.97% | 11.88% |

As can be seen from Table 2, the ability of the conventional second generation CAR-T cells targeting a classical tumor stem cell target EGFR vIII to kill glioma stem cells was weaker than that of the T cells expressing the truncated body of IL7Rα provided by the disclosure.

The preferred embodiments of the disclosure are described in detail above with reference to the accompanying drawings. However, the disclosure is not limited to the specific details in the above embodiments. Various simple variations may be made to the technical solutions of the disclosure within the scope of the technical idea of the disclosure, and these simple variations fall within the scope of the disclosure.

It should be further noted that the specific technical features described in the above specific embodiments may be combined in any suitable manner without contradiction. In order to avoid unnecessary repetition, the disclosure will not describe various possible combinations.

Moreover, the various different embodiments of the disclosure may be combined randomly without deviating from the idea of the disclosure, and the combinations should also be regarded as the disclosure of the disclosure.

## Claims

1. A truncated body of IL7Rα, wherein the amino acid sequence of the truncated body of IL7Rα comprises a sequence shown in SEQ ID NO. 1.

2. A nucleic acid, wherein the nucleic acid encodes the truncated body of IL7Rα according to claim 1; and preferably, the nucleic acid has a nucleotide sequence shown in SEQ ID NO. 2.

3. A fusion protein, wherein the fusion protein contains an antigen binding domain, a transmembrane domain and an intracellular signaling domain that are sequentially linked; the amino acid sequence of the intracellular signaling domain comprises the amino acid sequence of the truncated body of IL7Rα according to claim 1;

    preferably, the antigen binding domain comprises an anti-CD44 single-chain antibody and/or an anti-CD133 single-chain antibody, and the intracellular signaling domain comprises the truncated body of IL7Rα; and more preferably, the fusion protein has an amino acid sequence shown in SEQ ID NO. 3.

4. A fusion nucleic acid, wherein the fusion nucleic acid encodes the fusion protein according to claim 3; and preferably, the fusion nucleic acid has a nucleotide sequence shown in SEQ ID NO. 4.

5. An expression vector, wherein the expression vector is inserted with an expression cassette, the expression cassette comprises a first nucleic acid fragment encoding an antigen binding molecule and a second nucleic acid fragment encoding an intracellular signaling molecule, the intracellular signaling molecule contains the truncated body of IL7Rα according to claim 1, and an IRES element or a 2A peptide encoding sequence is inserted between the first nucleic acid fragment and the second nucleic acid fragment; and preferably, the expression cassette is the fusion nucleic acid according to claim 4.

6. A cell expressing a chimeric antigen receptor, wherein the cell expressing a chimeric antigen receptor is obtained by transfection of the expression vector according to claim 5 by a host cell, and the chimeric antigen receptor contains the truncated body of IL7Rα according to claim 1.

7. The cell according to claim 6, wherein the host cell is a T cell.

8. Use of the truncated body of IL7Rα according to claim 1, the nucleic acid according to claim 2, the fusion protein according to claim 3, the fusion nucleic acid according to claim 4, the expression vector according to claim 5, and the cell expressing a chimeric antigen receptor according to claim 6 or 7 in preparation of a medication for treating a tumor.

9. The use according to claim 8, wherein the tumor is a glioma.

10. A pharmaceutical composition, wherein an active ingredient of the pharmaceutical composition comprises the cell expressing a chimeric antigen receptor containing the truncated body of IL7Rα according to claim 6 or 7.

Fig. 1

Fig. 2

Fig. 3

Fig. 4

**EP 4 190 807 A1**

<table>
<tr><td colspan="2"><b>INTERNATIONAL SEARCH REPORT</b></td><td>International application No.<br><b>PCT/CN2021/109864</b></td></tr>
</table>

**A. CLASSIFICATION OF SUBJECT MATTER**

C07K 14/715(2006.01)i; C12N 15/12(2006.01)i; C07K 19/00(2006.01)i; C12N 15/62(2006.01)i; C12N 15/867(2006.01)i; C12N 5/10(2006.01)i; A61K 39/00(2006.01)i; A61P 35/00(2006.01)i

According to International Patent Classification (IPC) or to both national classification and IPC

**B. FIELDS SEARCHED**

Minimum documentation searched (classification system followed by classification symbols)

C07K; C12N; A61K; A61P

Documentation searched other than minimum documentation to the extent that such documents are included in the fields searched

Electronic data base consulted during the international search (name of data base and, where practicable, search terms used)

CNABS; VEN; CNKI; NCBI; CNTXT; EPTXT; USTXT; WOTXT; ISI web of science和关键词: 嵌合抗原受体, 截短, 融合, chimeric antigen receptor, CAR, IL7Rα, CD127 , truncat+, fus+等 China Patent Biological Sequence Search System; Genbank; EMBL and sequences searched: SEQ ID NOs: 1-8

**C. DOCUMENTS CONSIDERED TO BE RELEVANT**

| Category* | Citation of document, with indication, where appropriate, of the relevant passages | Relevant to claim No. |
|---|---|---|
| A | CN 110564695 A (EAST CHINA NORMAL UNIVERSITY et al.) 13 December 2019 (2019-12-13)<br>see entire document | 1-10 |
| A | CN 109503716 A (SHENGYAN MEDICINE TECHNOLOGY WUHAN CO., LTD.) 22 March 2019 (2019-03-22)<br>see entire document | 1-10 |
| A | CN 110526987 A (SHENZHEN BINDE BIOTECHNOLOGY CO., LTD.) 03 December 2019 (2019-12-03)<br>see entire document | 1-10 |
| A | WO 2017029512 A1 (AUTOLUS LTD.) 23 February 2017 (2017-02-23)<br>see entire document | 1-10 |
| A | WO 2018078066 A1 (MILTENYI BIOTEC GMBH) 03 May 2018 (2018-05-03)<br>see entire document | 1-10 |
| A | WO 2014124143 A1 (ANTHROGENESIS CORP.) 14 August 2014 (2014-08-14)<br>see entire document | 1-10 |

☐ Further documents are listed in the continuation of Box C.     ☑ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **22 September 2021** | **08 October 2021** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**

International application No.

**PCT/CN2021/109864**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a. ☑ forming part of the international application as filed:

        ☑ in the form of an Annex C/ST.25 text file.

        ☐ on paper or in the form of an image file.

    b. ☐ furnished together with the international application under PCT Rule 13*ter*.1(a) for the purposes of international search only in the form of an Annex C/ST.25 text file.

    c. ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**
Information on patent family members

| International application No. |
| --- |
| **PCT/CN2021/109864** |

| Patent document cited in search report | | | Publication date (day/month/year) | Patent family member(s) | | | Publication date (day/month/year) |
| --- | --- | --- | --- | --- | --- | --- | --- |
| CN | 110564695 | A | 13 December 2019 | None | | | |
| CN | 109503716 | A | 22 March 2019 | CN | 109503716 | B | 27 April 2021 |
| | | | | US | 2021000870 | A1 | 07 January 2021 |
| CN | 110526987 | A | 03 December 2019 | None | | | |
| WO | 2017029512 | A1 | 23 February 2017 | MX | 2019013204 | A | 21 January 2020 |
| | | | | US | 10800854 | B2 | 13 October 2020 |
| | | | | US | 10800855 | B2 | 13 October 2020 |
| | | | | KR | 20180054625 | A | 24 May 2018 |
| | | | | US | 2018244797 | A1 | 30 August 2018 |
| | | | | CN | 108350047 | A | 31 July 2018 |
| | | | | MX | 2018002042 | A | 19 June 2018 |
| | | | | AU | 2016308618 | A1 | 08 March 2018 |
| | | | | SG | CN 10201909759 U | A | 28 November 2019 |
| | | | | EP | 3337814 | A1 | 27 June 2018 |
| | | | | JP | 2021035395 | A | 04 March 2021 |
| | | | | CN | 110724200 | A | 24 January 2020 |
| | | | | JP | 2019150066 | A | 12 September 2019 |
| | | | | RU | 2018109350 | A3 | 09 December 2019 |
| | | | | BR | 112018003308 | A2 | 18 September 2018 |
| | | | | IL | 257234 | D0 | 29 March 2018 |
| | | | | CL | 2018000415 | A1 | 20 July 2018 |
| | | | | CA | 2995785 | A1 | 23 February 2017 |
| | | | | HK | 1252161 | A1 | 17 May 2019 |
| | | | | AU | 2016308618 | B2 | 13 May 2021 |
| | | | | CA | 3091907 | A1 | 23 February 2017 |
| | | | | EP | 3438123 | A1 | 06 February 2019 |
| | | | | JP | 2018523485 | A | 23 August 2018 |
| | | | | US | 2019016820 | A1 | 17 January 2019 |
| | | | | RU | 2018109350 | A | 23 September 2019 |
| | | | | US | 2021040227 | A1 | 11 February 2021 |
| | | | | CL | 2019001107 | A1 | 05 July 2019 |
| | | | | GB | 201514875 | D0 | 07 October 2015 |
| | | | | US | 2021040228 | A1 | 11 February 2021 |
| WO | 2018078066 | A1 | 03 May 2018 | US | 2019315879 | A1 | 17 October 2019 |
| | | | | EP | 3315511 | A1 | 02 May 2018 |
| | | | | CN | 109906231 | A | 18 June 2019 |
| | | | | EP | 3532490 | A1 | 04 September 2019 |
| WO | 2014124143 | A1 | 14 August 2014 | AU | 2018203558 | B2 | 01 October 2020 |
| | | | | SG | 10201810087 Q | A | 28 December 2018 |
| | | | | JP | 6467661 | B2 | 13 February 2019 |
| | | | | JP | 2021072827 | A | 13 May 2021 |
| | | | | US | 2018273640 | A1 | 27 September 2018 |
| | | | | AU | 2014214850 | A1 | 13 August 2015 |
| | | | | CA | 2904014 | A1 | 14 August 2014 |
| | | | | ES | 2748398 | T3 | 16 March 2020 |
| | | | | CN | 105101803 | A | 25 November 2015 |
| | | | | EP | 2953475 | A4 | 02 November 2016 |
| | | | | SG | 11201507026 W | A | 29 October 2015 |
| | | | | EP | 3604500 | A1 | 05 February 2020 |

Form PCT/ISA/210 (patent family annex) (January 2015)

**INTERNATIONAL SEARCH REPORT**
Information on patent family members

International application No.

**PCT/CN2021/109864**

| Patent document cited in search report | Publication date (day/month/year) | Patent family member(s) | | | Publication date (day/month/year) |
|---|---|---|---|---|---|
| | | NZ | 711807 | A | 26 June 2020 |
| | | AU | 2014214850 | B2 | 14 June 2018 |
| | | JP | 2019062902 | A | 25 April 2019 |
| | | JP | 2016506746 | A | 07 March 2016 |
| | | HK | 1254962 | A1 | 02 August 2019 |
| | | EP | 2953475 | A1 | 16 December 2015 |
| | | AU | 2020294311 | A1 | 18 February 2021 |
| | | AU | 2018203558 | A1 | 07 June 2018 |
| | | US | 2015368360 | A1 | 24 December 2015 |
| | | CN | 107988165 | A | 04 May 2018 |
| | | KR | 20150118977 | A | 23 October 2015 |
| | | CA | 3115383 | A1 | 14 August 2014 |
| | | HK | 1218049 | A1 | 03 February 2017 |
| | | NZ | 750426 | A | 26 June 2020 |
| | | EP | 2953475 | B1 | 03 July 2019 |
| | | AU | 2014214850 | C1 | 06 December 2018 |

Form PCT/ISA/210 (patent family annex) (January 2015)